Europäisches Patentamt

(19) European Patent Office

Office européen des brevets

(11) Publication number: **0 139 363**
**A1**

## (12) EUROPEAN PATENT APPLICATION

(21) Application number: **84305244.0**

(22) Date of filing: **01.08.84**

(51) Int. Cl.⁴: **A 61 M 16/00**
**A 61 M 16/20**

(30) Priority: 02.08.83 US 519754
21.09.83 CA 437255
21.09.83 CA 437254
28.09.83 US 536539

(43) Date of publication of application:
02.05.85 Bulletin 85/18

(84) Designated Contracting States:
AT BE CH DE FR GB IT LI LU NL SE

(71) Applicant: **O-TWO SYSTEMS INTERNATIONAL INC.**
**7575 Kimbel Street**
**Mississauga Ontario, L5S 1C8(CA)**

(72) Inventor: **Flynn, Stephen D.**
**255 Chartwell Road**
**Oakville Ontario, L6L 3Z7(CA)**

(74) Representative: **Connor, Terence Kevin et al,**
**FITZPATRICKS Kern House 61/62 Lincoln's Inn Fields**
**London WC2B 6EX(GB)**

(54) Breating apparatus.

(57) A breathing valve (10) having a valve chamber (16) with
a two-way opening (36) for exit of exhaled gases and through
which atmospheric air may be drawin in, a valve member
(40) in the chamber (16), a one-way flow opening (54) in the
valve member (40) through which gas may flow from the
chamber (16) to a patient for breathing, and preventing
return flow in the opposite direction, a movable valve closure
forming part (44,48) of the valve member (40) movable into
and out of sealing engagement with the two-way opening
(36), and a pressure responsive device (50) in the valve
member (40) responsive to obstruction of gas flow into the
chamber (16) and further responsive to inspiratory effort by a
patient to cause the valve member (40) to move away from
the two-way opening (36) to permit air to flow through in the
two-way opening (36) into the chamber (16) for inhalation.
Also disclosed is a manual resuscitation bag (80) for use with
such a valve (10) of football shape, with an inlet (86) at one
said end, and an outlet (88) at the other end, flod-rings
(94,96,98) in each end (86,88) of reduced thickness, may be
telescoped, ridges (104,106) between the fold-rings
(94,96,98), and the centre (84) of the bag (80) having a wall
thickness equal to the thickness of the ridges (104,106) and
having longitudinal ribs (104) on the outer surface of the
centre (84) of the bag (80) providing increased resiliency.

Fig . 1.

EP 0 139 363 A1

0139363

1.

DESCRIPTION

BREATHING APPARATUS

The invention relates to a breathing apparatus having a breathing valve for resuscitation, and a manual resuscitator bag used with such a valve.

BACKGROUND OF THE INVENTION

When gases are administered to a patient, a valve is used to supply gas to the mask. The purpose of the valve is to allow gas to flow in when the patent breathes in and, when the patient exhales, the valve reverses, closing off gas flow and allows the exhaled air and gas to flow out to atmosphere. The patient may be unable to breath at all, or may be breathing spontaneously but with difficulty. The valve must therefore be capable of permitting spontaneous breathing, and also permit an assistant to carry out resuscitation.

One form of such a valve is shown in U.S. Letters Patent No. 4,071,025.

Such valves are usually used with a manual resuscitator bag, or bellows-like device. One form of such air bag is shown in U.S. Letters Patent 3,363,833.

One of the disadvantageous features of the bag described in the above-mentioned patent is that due to its thin-wall construction, it tended to collapse too easily. Once collapsed, the bag then took a considerable length of time to recover to its normal shape. There are no internal springs in such bag for various reasons , and it must rely on its own inherent resiliency to recover its original shape. Until it has recovered its own original shape, it is not possible for the operator to compress it again to create a further positive pressure for the patient. During normal non-emergency breathing assistance, the bag will not be compressed more than once for each breath of the patient. Since the normal patient will not inhale more frequently than about thirty to forty inhalations per minute, and provided the recovery time for the bag is no longer than about 1/2 second in length, then there is no problem. However, during emergency resuscitation, when a person is not breathing, the best medical practice recommends that four or five short, sharp pulses of positive pressure should be applied rapidly in quick succession. These initial pulses may be in the order of two or three per second. The slow recovery rate of the bag described in such patent therefore rendered it difficult if not impossible to apply these initial rapid pressure pulses.

It has been found by experience that the great majority of operators greatly prefer a bag which has an almost immediate recovery rate. This removes a source of worry or tension, and leaves the operator free to concentrate on the condition of the patient. This in turn, leads to a more carefully controlled and relaxed application of pressure in each cycle, which leads to an improved resuscitation effect, on the patient.

Equipment of this type is frequently used by paramedical personnel, nursing assistants, first aid workers, firemen, and service personnel. Such equipment is frequently required in an emergency, and in situations where malfunction of the equipment can occur for various reasons. For example, the gas supply can become blocked, due to a bend in a gas supply pipe for example. Similarly, a person being resuscitated is likely to vomit, and the vcmit will enter the mask and may enter the valve itself.

Numerous other emergencies can also occur.

In many of these situations the result is that the valve becomes blocked, or gas flow from the gas supply into the valve is cut off.

In these situations, the assistant administering the gas may be distracted, and may not notice what has occurred. The patient is then in danger of suffocating.

Accordingly, it is desirable to provide in such a valve a means whereby in the event of a breakdown in the gas supply for any reason, atmospheric air is admitted to the valve, so that the patient can continue breathing.

Usually, the blocked valve or blocked gas supply condition will last only a few seconds at most, depending upon the experience of the assistant administering the gas. Once the assistant notices the problem, the mask is then quickly removed and either the valve must be dismantled and washed out, or the blockage in the supply must be cleared, and the valve and mask are then reassembled to continue treatment.

A somewhat improved form of valve is shown in Canadian Letters Patent No. 798,660. The valve shown in that patent involves two separate flexible valve members. One valve member opens to permit inhalation of gases, while the other valve member closes against the introduction of atmospheric air. On exhalation, the inlet valve moves away, and the exhalation valve opens up, allowing exhaled air to escape to atmosphere.

This type of valve does not provide for inhalation of atmospheric air in the event of a breakdown in the supply of gas.

If the gas supply becomes blocked for some reason, the patient will be unable to inhale atmospheric air, and will essentially suffocate unless the mask is immediately removed.

Throughout this description, reference is made to the administering of a gas to a patient. It will of course be appreciated that the gas may be either pure oxygen, or air enriched with oxygen or ambient air, or a specially formulated mixture of gases.

For the purposes of this discussion therefore when reference is made to the administration of a gas, the term "gas" includes any mixture which may be breathed, including fresh air, oxygen, oxygen enriched air and specially formulated mixtures.

BRIEF SUMMARY OF THE INVENTION

With a view to overcoming these problems, the invention therefore comprises a valve for use in association with breathing devices for the administration of a gas, and having a valve chamber having an upstream end, connectable to a supply of gas, and having a downstream and connectable to a breathing device, there being respective upstream and downstream opening means in said chamber communicating with respective said connection means, whereby gas can be admitted through said upstream opening, and can be administered through said downstream opening, said chamber means being of two-part construction, and including releasable connection means whereby the same may be dismantled and reassembled, air flow opening means adjacent said downstream end, whereby exhaled air and gases may be discharged to atmosphere, and through which atmospheric air may be inducted into said chamber, a one-piece integral flexible valve member releaseably fastened in said chamber, one-way flow opening means in said valve member, whereby gas may flow from said chamber into said downstream opening, said valve member preventing return flow in the opposite direction, and said valve member further incorporating movable valve closure means movable into and out of sealing engagement with said air flow opening means in said downstream end of said chamber, said seating-means closing said openings, upon flow of gas from said upstream end of said chamber, and said seating means unsealing said air flow opening means upon exhalation, and means incorporated in said valve member normally holding said valve seating member away from said air flow openings, whereby upon the obstruction of gas flow into said chamber from said upstream end, air may flow through said air flow openings into said chamber for inhalation through said downstream end.

The invention also provides a resuscitator bag for use with the valve, the bag having a generally elongated football shape, inlet means at one end, and outlet means at other end, and each said end having fold-rings of reduced thickness, and ridges of greater thickness between such fold-rings whereby said end portions may be telescoped, and having a centre portion having a wall thickness substantially equal to the thickness of said ridges in the ends between said rings, and there being a plurality of longitudinal ribs formed on the outer surface of said centre portion providing increased resiliency.

6.

The various features of novelty which characterize the invention are pointed out with particularity in the claims annexed to and forming a part of this disclosure. For a better understanding of the invention, its operating advantages and specific objects attained by its use, reference should be had to the accompanying drawings and descriptive matter in which there are illustrated and described preferred embodiments of the invention.

IN THE DRAWINGS

Figure 1 is an elevational view of a typical gas administration system (without showing the bag or gas supply), and showing the valve according to the invention in section;

Figure 2 is a greatly enlarged sectional view of the valve shown in Figure 1, shown in the normal gas inhalation position;

Figure 3 is a perspective of the valve member;

Figure 4 is a sectional illustration corresponding to Figure 2, and showing either exhalation or intake of fresh air;

Figure 5 is a section of an alternate embodiment.

Figure 6 is a perspective illustration showing a bag and valve according to the invention and a typical mask;

Figure 7 is a section of the bag along the line 7-7;

Figure 8 is a section along the line 8-8 of Figure 6.

## DESCRIPTION OF A SPECIFIC EMBODIMENT

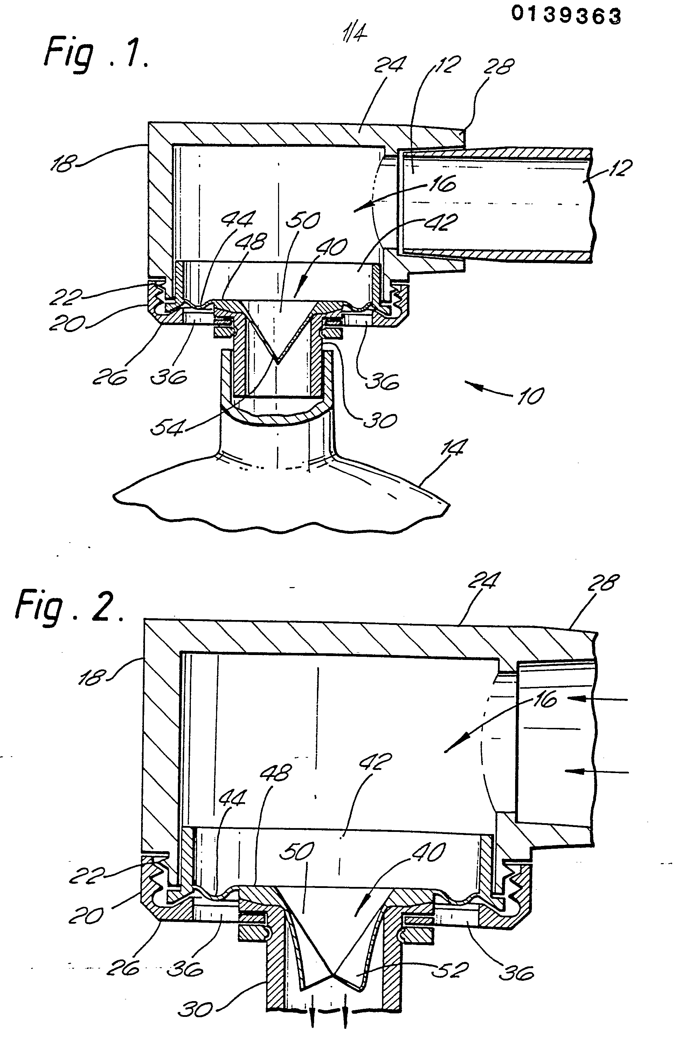

Referring now to Figure 1, a valve according to the invention is shown generally as 10. For the sake of explanation only, it is shown connected to a gas supply pipe 12, at its upstream end, and at its downstream end, it is shown connected to a typical breathing mask 14.

It will of course be appreciated that the gas supply 12 is merely exemplary of a variety of different forms of supply that may be used. Typically the gas supply will be atmospheric air, supplied by means such as an air bag, such as that shown in Figure 6, the details of which are described below.

The mask 14 could be any one of a wide variety of different masks, or other devices such as a breathing tube which is inserted down the windpipe.

Valve 10 will be seen to comprise a chamber indicated generally as 16, which is a two-part structure having an upper generally cylindrical wall 18, and a lower generally cylindrical wall 20. Threads 22 fasten walls 18 and 20 together.

An upstream end wall 24 closes off the upper end of cylindrical portion 18, and a downstream end wall 26 closes off the downstream end of lower cylindrical wall 20.

A friction fit sleeve 28 provides a coupling with gas supply pipe 12. A downstream connector 30 having a retaining flange 32 is rotatably received in a central opening in wall 26. A snap-ring 34 secures connector 30 in position. Connector 30 is of a standard diameter to connect with other breathing devices.

Other forms of connection may be provided in various different circumstances without altering the invention.

It will also be noted that flange 32 has a raised sealing ridge 35, for reasons described below.

Downstream end wall 26 is provided with a plurality of air flow openings 36, for discharging exhaled air and gas, and also for admitting fresh air if an emergency blockage occurs.

The walls of valve chamber 16 are generally formed of clear plastic materials so that the interior of the chamber is readily visible. A releaseable locking device (not shown) can be provided for threads 22 if desired.

Within valve chamber 16, there is provided the one-piece integral valve member or element 40. Valve member 40 has a generally annular outer skirt portion 42 which makes a sliding fit within the interior of upper cylindrical wall 18. Skirt 42 assists in locating the valve member centrally in the chamber, and also prevents assembly of the valve member in any orientation other than the correct one as shown.

A peripheral retaining flange 43 extends outwardly past the lowermost portion of wall 18, to retain valve member 40 in position.

An annular flexible web 44 extends inwardly from skirt 42. Web 44 is dimensioned and oriented to overlay air flow openings 36, and is thin and flexible for reasons described below.

Located inwardly of closure portion 44, is a more rigid annular seating ring 46, dimensioned to seat on sealing ridge 35 of flange 32. A tubular member 50 extends from the inner extremity of ring 46, and is designed to extend down the interior of connection portion 30. The tubular member 50 is essentially a pair of inwardly tapered flexible lips 52. Lips 52 meet one another at a slit 54, which is normally closed by the inherent inward bias of the lips 52.

It will be appreciated that valve member 40 is of integral one-piece construction, being formed of flexible resilient material such as synthetic rubber materials, typically silicone based plastic materials.

The valve is usually used in conjunction with bag 80, shown in Figures 6, 7 and 8. Bag 80 will usually have an air intake valve 82. In cases where it is used in conjunction with a breathing gas supply, such a gas supply can be connected by means such as the hose H shown in phantom.

The gas supply may include a gas accumulator (not shown) of well-known design for collecting gas and storing it at or about atmospheric pressure.

The bag 80 is typically of such a size that it may be operated with one hand, and squeezed and released, or alternatively may lie on any available surface, so that an operator may simply press down on the bag.

Usually, there is some form of flexible or swingable or rotatable connection between the bag 80 and the breathing valve 10, and if necessary, a longer piece of hose can be incorporated.

For the purpose of this explanation, the bag 80 may be considered as having a central portion 84 of maximum diameter, and two progressively tapering end portions 86-88, terminating in end connector collars 90-92, giving it a football shape.

The end portions 86 and 88 are intended to be infolded in a telescoping manner within the central portion 84, for packing and storage.

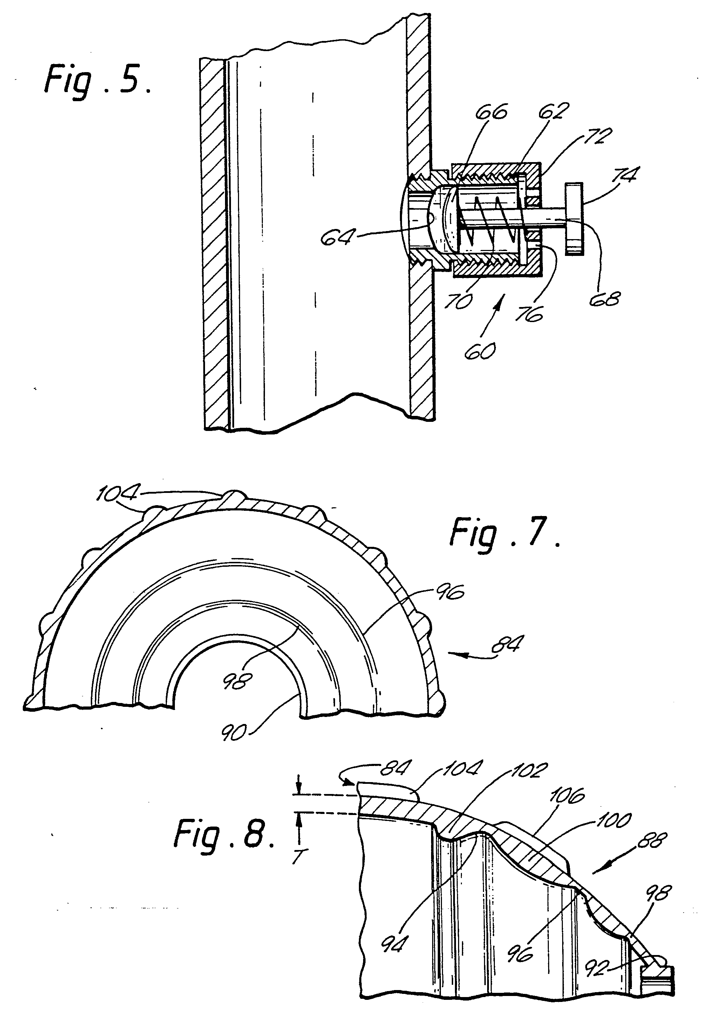

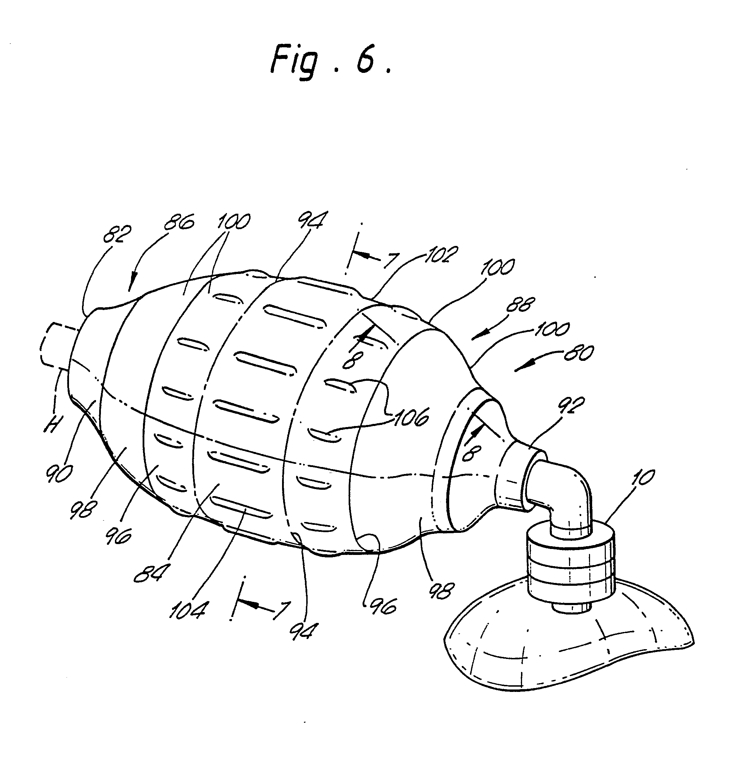

In order to accommodate this function, the end portions are formed as shown in Figure 8. Each end has three reduced thickness fold rings 94, 96 and 98. The ring 94 is of greater diameter and the ring 96 of lesser diameter. Thickened ridge portions 100 extend between the rings. The rings permit the end portions 86 and 88 to be retracted in a generally telescoping manner. When required for use, the end portions 86-88 may be readily snapped out simply by pulling on the end connectors 90-92.

In order to provide the bag with a substantial degree of inherent resilience, the central portion 84 is made with a wall thickness shown as T, which is essentially equal to the wall thickness of the ridge portions 100 of the tapered ends 86-88. In addition, a thickened reinforcing ring 102 is provided at the transition from the central portion 84, and the first ring 94.

Additional longitudinal reinforcing ribs 104 are provided on the exterior of the central portion 84 aligned with the central axis of the bag 80, and arranged at spaced intervals. The spring ribs 104 extend substantially from one of the internal reinforcing rings 102 to the other, along the full longitudinal extent of the central portion 84. The spring ribs 104 function to provide an effective outward springing action to the bag, causing it to rapidly recover its original shape after it has been compressed.

11.

Additional spring ribs 106 are provided on the tapering end portions 86-88. The spring ribs 106 preferably extend over the larger reduced thickness ring 94, at spaced intervals therearound.

The spring ribs 104 and 106 are moulded integrally in one piece of thermoplastic material as shown, and being of the same material, consequently have the same extended storage life and resistance to deterioration.

The usage of the bag 80 is self evident. In use it will normally be compressed progressively to induce inhalation. After an inhalation stroke, the bag 80 is released and allowed to recover its original shape. During this phase pressure may be applied to the chest or abdomen of the patient, causing him to exhale. In the normal case exhalation takes place through openings 36 in the breathing valve 10.

During the initial phases of resuscitation, particularly where an emergency has occurred and the patient is not breathing, four or five rapid pulses are usually administered. This is achieved by rapidly compressing and releasing the bag 80. It is found that the bag 80 performs well during this rapid initial pulsing, and provides the virtually instantaneous recovery of the bag shape which is required for this type of treatment.

Where the bag is used to resuscitate an infant, the breathing rate may be much higher, i.e., sixty breaths per minute. It is not found that there is any increased danger of overpressuring the lungs, when using the bag, and in fact, the rapid recovery rate reduces operator stress and enables him to concentrate on controlling the treatment.

With reference to the operation of the valve, gas (or air) is supplied under a slight positive pressure, e.g., about 2 mm of mercury, through pipe 12, and will fill chamber 16. Flow of gas through the chamber 16 is however impeded or checked by the valve member 40. The inherent bias of lips 52 is sufficiently strong to overcome the slight pressure at this stage.

Where the patient is unable to breathe, then positive pressure is applied, i.e., by hand pressure on the bag 80. The increased pressure in chamber 16 will then cause lips 52 to open and force gas or air into the lungs of the patient.

Where the patient is breathing spontaneously, the patient inhales and a slight negative pressure will then be developed in the mask 14.

This will then increase the pressure differential across the valve member 40. The inherent bias of lips 52 will then yield to the greater pressure differential, allowing gas to flow through the slit opening 54 as shown in Figure 2. Pressure on the bag is then released.

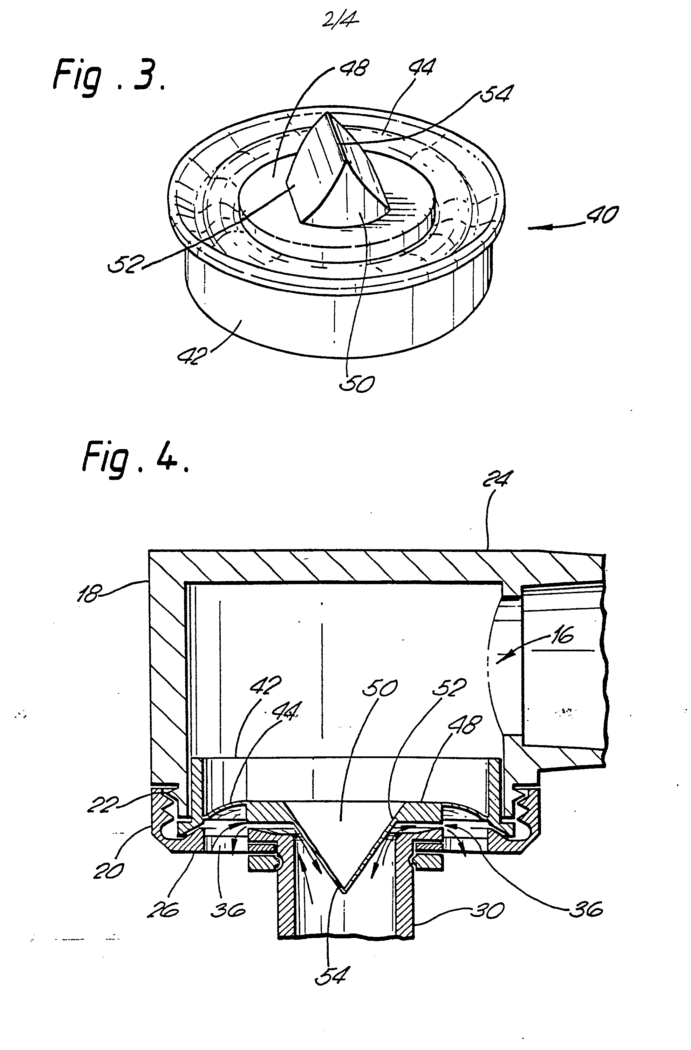

As the patient then exhales either consciously or unconsciously, a slight positive pressure is created in mask 14. This positive pressure will then cause valve member 40 to flex slightly upwardly as shown in Figure 4. Web 44 will flex and permit ring 46 to lift off sealing ridge 35. Simultaneously, this will cause the tubular member 50 to move up within connector 30. This will create an outward flow path for exhaled air which can then flow up around the tubular member 50, over ridge 35, and out to atmosphere through openings 36.

If a malfunction occurs, and gas flow in pipe 12 is cut off, and the patient then attempts to inhale, a negative pressure is created within mask 14. In the absence of a slight positive pressure from the gas flow in chamber 16, the valve member 40 and particularly web 44 will then function as a diaphragm. It will flex upwardly into chamber 16, in an attempt to equalize the pressure throughout the system and ring 46 will lift off ridge 35. At the same time, since the pressure within mask 14 is at this point slightly below atmospheric, air will attempt to flow inwardly through openings 36. Because the valve member 40 has flexed inwardly into chamber 16, it will open up a slight spacing around tubular member 50, permitting such outside air to flow inwardly, substantially in the same position as shown in Figure 4, so that the patient can then continue to breathe normally.

If the patient should vomit into the mask, and if any vomit should pass over into the valve member 16, the mask can instantly be connected from the valve chamber, and the valve chamber can be removed from the gas supply pipe 12. The mask can then simply be washed or wiped clean. The valve chamber 16 can be opened up by a simple rotary unthreading action. The valve member 40 can be removed and washed. If when it is reassembled, the assistant attempts to assemble the valve member 40 the wrong way around, the skirt 42 will interfere so that the assistant will know that the valve member 40 is not in the correct position.

In the case of the treatment of infants it may be additionally desirable to provide a pressure relief valve. Such a relief valve is shown generally as 60 and has a body 62, and seat 64. A valve disc 66 is located on a rod 68. Spring 70 urges disc 66 onto seat 64. A threaded cap 72 retains spring 70 and permits adjustment of spring pressure. A finger button 74 permits holding

14.

of the disc 66 on seat 64 if it is desired to override the spring. Openings 76 in cap 72 permit release of gas during overpressure.

The foregoing is a description of a preferred embodiment of the invention which is given here by way of example only. The invention is not to be taken as limited to any of the specific features as described, but comprehends all such variations thereof as come within the scope of the appended claims.

15.

## CLAIMS

1. A breathing system for use in association with breathing devices for the administration of gases to a patient, and comprising: a valve chamber having an upstream end connectable to a supply of gas, and having a downstream end connectable to a breathing device, there being respective upstream and downstream opening means in said chamber communicating with respective said connection means, whereby gas can be admitted through said upstream opening, and can be administered through said downstream opening; gas flow opening means adjacent said downstream end whereby exhaled gases may be discharged to atmosphere, and through which atmospheric air may be inducted into said chamber; a valve member extending across such chamber between said upstream and downstream ends; one-way flow opening means in said valve member whereby gas may flow from said chamber into said downstream opening, said valve member preventing return flow in the opposite direction; movable valve closure means forming part of such valve member movable into and out of sealing engagement with said gas flow opening means in said downstream end of said chamber, whereby said closure means will close said openings, upon inhalation of gas from said upstream end of said chamber, and said closure means unsealing said gas flow opening means upon exhalation; pressure responsive means incorporated in said valve member responsive to obstruction of gas flow into said chamber from said upstream end, and further responsive to inspiratory effort by a patient whereby to cause said valve member to move away from said gas flow openings, thereby permitting air flow through said gas flow openings into said chamber for inhalation through said downstream end, and, gas supply means for supplying gas under pressure to said upstream end of said valve chamber.

16.

2.    A breathing system as claimed in Claim 1 wherein such valve member incorporates a relatively rigid annular ring portion formed integrally therewith, and a flexible diaphragm portion.

3.    A breathing system as claimed in Claim 2 in which the valve member has interference means to prevent assembly the wrong way around.

4.    A breathing system as claimed in Claim 1 wherein such valve chamber is of two part construction, and including releasable fastening means for fastening the two parts of the valve chamber together, whereby it may readily be disassembled and reassembled for cleaning.

5.    A breathing system as claimed in Claim 1 wherein such downstream opening means, and downstream connection means, comprise a tubular member, a flange formed at an upper end of such tubular member, valve seating surfaces defined by such upper end of said tubular member, an opening in said valve chamber through which said member passes, said opening means being smaller than said flange, whereby said tubular member is retained in said chamber with a portion thereof extending upwardly through said opening, and retaining means on said tubular member outwardly of said chamber, preventing the same from being forced into said chamber, said tubular member being rotatable relative to said chamber in said opening to facilitate manipulation of the device in use in various positions.

6.    A breathing system as claimed in Claim 5, wherein said gas flow opening means are located adjacent said opening for receiving said tubular member.

7.    A breathing system as claimed in Claim 6 wherein said valve member incorporates a relatively rigid annular ring portion formed integrally therewith, dimensioned and arranged to contact with said flange of said tubular member.

8. A breathing system as claimed in Claim 7 wherein said valve member further incorporates an elongated tapering extension portion located inwardly of said rigid annular member, and extending downwardly into the upper end of said tubular member, said tapering extension defining two flexible lip portions, arranged so as to normally contact one another at their tips, and being movable away from one another to permit one way flow of gas therethrough into said tubular member.

9. A breathing system as claimed in Claim 8 including a flexible diaphragm portion forming part of said valve member, located outwardly of said rigid annular portion, and an outer peripheral generally cylindrical wall portion located outwardly of said flexible diaphragm portion, said flexible diaphragm portion overlying said exhalation openings.

10. A breathing system as claimed in Claim 1 incorporating pressure relief valve means located in communication with said upstream end of said chamber, and, manual override means for overriding such pressure relief valve.

11. A breathing system as claimed in Claim 1 wherein said breathing device includes a manually operable resuscitator bag of one piece generally elongated football shape, defining an enlarged centre portion of maximum diameter, and two tapering end portions of progressively reducing diameter; inlet means at one said end, and outlet means at the other said end; fold-rings in each said tapering end portion comprising wall portions of reduced thickness, defining flex zones in said tapering end

Fig.1.

Fig.2.

0139363

Fig .3.

Fig . 4.

0139363

3/4

**Fig .5.**

66  62  72  74
64  70  76  68  60

**Fig .7.**

104  96  84  98  90

**Fig .8.**

84  104  102  106  100  88  98
T  94  96  92

Fig. 6.

0139363

4/4

| Category | Citation of document with indication, where appropriate, of relevant passages | Relevant to claim | CLASSIFICATION OF THE APPLICATION (Int Cl.4) |
|---|---|---|---|
| | **DOCUMENTS CONSIDERED TO BE RELEVANT** | | EP 84305244.0 |
| X | DE - B2 - 1 791 014 (OX-VITAL L. MICZKA KG) | 1,4 | A 61 M 16/00 |
| A | * Totality * | 5,6 | A 61 M 16/20 |
| | -- | | |
| D,X | DE - B - 1 616 422 A.S. LAERDAL) | 1,4 | |
| Y | * Totality * | 10 | |
| A | | 2,5,6, 8,9 | |
| | -- | | |
| Y | US - A - 4 192 301 (H.W. HARDWICK) | 10 | |
| | * Fig. 2; column 2, lines 46-59 * | | |
| | -- | | |
| A,D | DE - B - 1 616 421 (A.S. LAERDAL) | 1,5,8, 11 | |
| | * Totality * | | |
| | -- | | **TECHNICAL FIELDS SEARCHED (Int. Cl.4)** |
| A | US - A - 3 009 459 (H. RUBEN) | 1,11 | A 61 M 16/00 |
| | * Fig. 3; column 2, lines 37-47 * | | |
| | -- | | |
| A | FR - E - 1 183 803 (TESTA LAB.) | 1,11 | |
| | * Fig. 1; page 2, lines 8-22 * | | |
| | -- | | |
| D,A | US - A - 4 071 025 (R.L. HESSE) | 1,2,4, 5,11 | |
| | * Totality * | | |
| | ---- | | |

The present search report has been drawn up for all claims

| Place of search | Date of completion of the search | Examiner |
|---|---|---|
| VIENNA | 09-01-1985 | LUDWIG |